# EUROPEAN PATENT SPECIFICATION

(11) **EP 4 171 505 B1**
(45) Date of publication and mention of the grant of the patent: **10.12.2025**
(21) Application number: 21732350.0
(22) Date of filing: 22.06.2021
(51) Int. Cl.: A61K 8/81, A61K 8/49, A61Q 5/00

(54) **HAIR CARE COMPOSITION**
HAARPFLEGEZUSAMMENSETZUNG
COMPOSITION DE SOINS CAPILLAIRES

(30) Priority: 25.06.2020 EP 20182357
(43) Date of publication of application: 03.05.2023
(73) Proprietor: Unilever IP Holdings B.V., 6708 WH Wageningen (NL); Unilever Global IP Limited, Wirral, Merseyside CH62 4ZD (GB)
(72) Inventor: AINGER, Nicholas, John, Wirral Merseyside CH63 3JW (GB); COLLINS, Luisa, Zoe, Wirral Merseyside CH63 3JW (GB); DAWSON, Joanna, Susan, Wirral Merseyside CH63 3JW (GB); ROBERTS, Louise, Jannette, Wirral Merseyside CH63 3JW (GB); WHITEHEAD, Paul, Stephen, Wirral Merseyside CH63 3JW (GB)
(74) Representative: Oates, Elizabeth Ellen
(86) International application number: PCT/EP2021/067029
(87) International publication number: WO 2021/259943

(56) References cited:
- WO-A1-2018/007332
- US-A1- 2008 206 355
- US-A1- 2019 328 647
- ANONYMOUS: "Ashland brings enduring dimension to hair repair with the introduction of N-DurHance A-1000 conditioning polymer", INTERNET CITATION, 4 January 2014 (2014-01-04), pages 1 - 5, XP002757185, Retrieved from the Internet <URL:http://investor.ashland.com/releasedetail.cfm?releaseid=836948> [retrieved on 20160429]

## Description

### Field of the Invention

The present invention relates to a hair care composition, particularly an anti-dandruff shampoo composition.

### Background of the Invention

Hair care compositions generally provide cleansing or conditioning benefits or a combination of the two. Such compositions typically comprise one or more cleansing surfactants which generally aid in cleaning the hair and the scalp free of undesirable soil, particles and fatty matter.

Dandruff is a problem affecting many globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, hair treatment compositions are developed including various actives for their antidandruff effectiveness. Piroctone compound such as piroctone olamine is one such active.

A common problem with piroctone compound is that its deposition onto the hair or scalp during the wash process is difficult. This is particularly the case when effective anionic cleansing surfactants such as sodium laureth sulphate are present in the composition. During the excessive rinsing process, the majority of piroctone is likely to be washed away together with the surfactants. Poor deposition is correlated with low antidandruff activity, thus little mitigation of the ill-effects of dandruff. To date, there are attempts to offset this drawback by increasing the level of piroctone olamine in hair treatment composition. Such approach causes a variety of issues such as increased costs, potential instability of the formulation and potential adverse effect to hair sensory. Hence it is not an approach favoured by the industry.

Cationic polymers are used to enhance the deposition of conditioning agents and/or anti-dandruff agents onto the hair and/or scalp. These polymers may be synthetic or natural polymers that have been modified with cationic substituents.

US 2019/000735 describes hair care compositions having a 2-pyridinol-N-oxide material and an iron chelator to provide antifungal efficacy

The present invention relates to enhanced deposition of a piroctone compound (Octopirox) using cationic polymers with a defined charge density.

### Description of the Invention

In a first aspect, the present invention relates to a hair care composition according to claim 1.

### Detailed Description of the Invention

The term hair care composition refers to compositions for topical application to hair and/or scalp of mammals, especially humans. Such a composition may be generally classified as leave-on or rinse off. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a shampoo.

Cationic charge density refers to the number of cationic charges per weight unit of a given polymer. As used herein, the term "charge density" refers to the ratio of positive charges on a 20 monomeric unit of which a polymer is comprised to the molecular weight of said monomeric unit. The charge density multiplied by the polymer molecular weight determines the number of positively charged sites on a given polymer chain. Cationic charge density can be calculated from the degree of substitution as described in WO 2013/011122, page 8 lines 8-17.

The cationic charge density of the polymer may also suitably be determined via the Kjeldahl method as described in the US Pharmacopoeia under chemical tests for nitrogen determination and is expressed in milli-equivalents (meq) per gram.

The cationic charge density is determined at pH7.

Water-insoluble, refers to the solubility of a material in water at 25°C and atmospheric pressure being 0.1% by weight or less.

Molecular weight refers to the weight average molecular mass of a given polymer. The weight average molecular weight (WAVG MW) of a given polymer is determined by SEC (Size Exclusion Chromatography) analysis using absolute calibration (universal calibration). Polysaccharide standards pullulan and dextran were used for calibration. Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified.

It should be noted that in specifying any ranges of values, any particular upper value can be associated with any particular lower value.

Where a feature is disclosed with respect to a particular aspect of the invention (for example a composition of the invention), such disclosure is also to be considered to apply to any other aspect of the invention (for example a method of the invention) *mutatis mutandis.*

The polymer suitable for use in compositions of the present invention comprises acrylamidopropyltrimonium chloride. Preferably, the copolymer also comprises acrylamide in addition to the acrylamidopropyltrimonium chloride. Particularly preferred copolymer is a copolymer of acrylamidopropyltrimonium chloride and acrylamide.

The homopolymer and/or copolymer according to the present invention has a charge density has a charge density of at least 3.5 meq/g. more preferably of 4 meq/g or greater, most preferably from 4.3 to 5.3 meq/g at pH 7.

The homopolymer and/or copolymer preferably has a weight average molecular weight from 100,000 to 500,000, more preferably form 150,000 to 400,000.

An example of a suitable homopolymer is commercially available from Ashland under the trade name N-DurHance A-1000^{®} ((cationic homopolymer of acrylamidopropyl trimethyl ammonium chloride). An example of a suitable copolymer is commercially available from Ashland under the trade name N-DurHance AA2000^{®} (cationic copolymer of acrylamidopropyltrimonium chloride/acrylamide copolymer).

Typically, the cationic polymer is present at a level of from 0.01 to 5% by weight of the total composition, preferably from 0.05 to 2 wt%, more preferably from 0.1 to 1 wt%.

Additional cationic deposition polymers may be included in addition to the homopolymer / copolymer, but they are not preferred.

The preferred format of the composition is an antidandruff shampoo. The anti-dandruff shampoo may suitably comprise from 50 to 90%, preferably from 60-80% water by weight of the total shampoo.

### Cleansing Phase

The cleaning phase comprises one or more cleansing surfactants. The cleansing surfactants refer to those which act to cleanse hair and/or scalp. The total level of cleansing surfactants is preferably from 3 to 45 %, more preferably from 5 to 25 %, most preferably from 7 to 20% by weight of the total composition.

Preferably, the cleansing surfactant comprises an anionic surfactant. The anionic surfactant comprises ethoxylated alkyl sulphate anionic surfactant.

Preferred alkyl ether sulphates are those of formula (I):

R-O-(CH₂CH₂-O)ₙ-SO₃⁻M⁺ (I)

in which R is a straight or branched alkyl chain having 8 to 18 (preferably 12 to 18) carbon atoms; n is the average degree of ethoxylation and ranges from 0.5 to 3 (preferably from 1 to 3); and M is a solubilizing cation such as sodium, potassium, ammonium or substituted ammonium. An example is sodium lauryl ether sulphate (SLES). The most preferred example is SLES having an average degree of ethoxylate of **from 0.5 to** 3, preferably from 1 to 3.

The preferred level of such surfactant is from 2 to 20% by weight of the total composition, more preferably from 7 to 15wt%.

Other surfactants may be present in the composition, such as alkyl sulphates. Preferred alkyl sulphates are C₈₋₁₈ alkyl sulphate, more preferably C₁₂₋₁₈ alkyl sulphate, preferably in the form of a salt with a solubilising cation such as sodium, potassium, ammonium or substituted ammonium. Examples are sodium lauryl sulphate (SLS) or sodium dodecyl sulphate (SDS).

The cleansing phase may comprise one or more further anionic surfactants which are cosmetically acceptable and suitable for topical application to hair and/or scalp. Examples of further anionic surfactants include alkyl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions according to the present invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.lauric monoglyceride sodium sulphate, sodium lauryl sulphate, sodium laureth sulphate, sodium cocyl sulphate, sodium cocoyl isethionate and mixtures thereof.

The composition can include co-surfactants, to help impart aesthetic, physical or cleansing properties to the composition. The co-surfactant is preferably comprised in the cleansing phase of the composition. An example of a co-surfactant is a nonionic surfactant, which can be included in an amount ranging from 0.5 to 10%, preferably from 2 to 8%, more preferably from 1 to 5% by weight of the total composition.

For example, representative nonionic surfactants that can be included in the treatment compositions, preferably shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Other representative nonionic surfactants include mono- or di-alkyl alkanolamides. Examples include coco mono- or di-ethanolamide and coco monoisopropanolamide.

Further nonionic surfactants which can be included are the alkyl polyglycosides (APGs). Typically, the APG is one which comprises an alkyl group connected (optionally via a bridging group) to a block of one or more glycosyl groups. Preferred APGs are defined by the following formula (II):

R'O - (G)ₖ (II)

in which R' is a branched or straight chain alkyl group which may be saturated or unsaturated and G is a saccharide group. R' may represent a mean alkyl chain length of from about C₅ to about C₂₀. Preferably R' represents a mean alkyl chain length of from about C₈ to about C₁₂. Most preferably the value of R' lies between about 9.5 and about 10.5. G may be selected from C₅ or C₆ monosaccharide residues and is preferably a glucoside. G may be selected from the group comprising glucose, xylose, lactose, fructose, mannose and derivatives thereof. Preferably G is glucose. The degree of polymerisation, k, may have a value of from about 1 to about 10 or more; preferably, the value of k lies from about 1.1 to about 2; most preferably the value of m lies from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions (preferably shampoos) of the invention include the C₁₀-C₁₈ N-alkyl (C₁-C₆) polyhydroxy fatty acid amides, such as the C₁₂-C₁₈ N-methyl glucamides, as described for example in WO 92/06154 and US 5,194, 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 10 wt.%, preferably from 2 to 8, more preferably from 1 to 5 % by weight of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, in which the alkyl and acyl groups have from 8 to 22 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is an amidobetaine amphoteric surfactant of general formula (III): 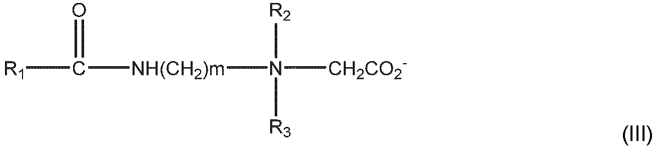 in which m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof. An example is cocamidopropyl betaine. The preferred level of such surfactant is from 0.5 to 10% by weight of the total composition, more preferably from 2 to 8 wt%, most preferably from 1 to 5 wt%.

A further optional but preferred surfactant is an alkyl glycinate and/or alkyl carboxyglycinate. If present, it is present at a level of from 1 to 8 wt.%, preferably 2 to 6 wt.%

Preferably the alkyl glycinate and/or alkyl carboxyglycinate has an alkyl group of from C₈₋₂₂ carbon atoms, in the form of a salt with a solubilising cation such as sodium, potassium, ammonium or substituted ammonium. Preferred glycinates are sodium coco glycinate and sodium cocoyl glycinate.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In preferred embodiments, the cleansing phase comprises an alkyl sulphate and/or ethoxylated alkyl sulphate anionic surfactant; and a betaine surfactant, preferably an alkyl amidopropyl betaine.

The total amount of surfactants (inclusive of any co-surfactants) in a hair treatment composition, is generally from 1 to 50 wt.%, preferably from 2 to 40 wt.%, more preferably from 10 to 25 wt.% by weight of the total composition.

### Piroctone Compound

The piroctone compound for use in the present invention may include piroctone acid, primary, secondary and tertiary olamine salts of piroctone acid (such as the diethanolamine and triethanolamine salts), and mixtures thereof, preferably piroctone acid, primary olamine salt of piroctone acid (i.e. piroctone olamine, also known as Octopirox^{®}) and mixtures thereof.

The piroctone compound useful in the present invention typically contains the structure defined by formula (IV): 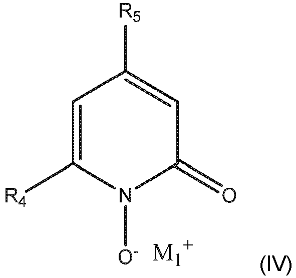
wherein R₄ is selected from C1-C17 hydrocarbon radicals, R₅ is selected from C1-4 alkyl, C2-4 alkenyl or alkynyl, hydrogen, phenyl or benzyl, and M₁ is selected from hydrogen, monoethanolamine (MEA), diethanolamine (DEA), or triethanolamine (TEA). Preferred R₄ group is (CH₃)₃CCH₂CH(CH₃)CH₂- and preferred R₅ is a methyl. More preferably, R₄ is
(CH₃)₃CCH₂CH(CH₃)CH₂-, R₅ is a methyl and M₁ is a hydrogen or MEA. Most preferably, R₄ is (CH₃)₃CCH₂CH(CH₃)CH₂-, R₅ is a methyl and M₁ is hydrogen.

Piroctone olamine is particularly preferred.

The typical level of the piroctone compound is from 0.01 to 5% by weight of the total composition, preferably from 0.05 to 2 wt%, more preferably from 0.1 to 1.5 wt%.

Preferably the weight ratio of homopolymer / copolymer to piroctone compound is preferably 2:1 or more preferably 1.5:1 or below, most preferably form 0.3:1 to 1.1:1.

Preferably piroctone compound forms at least 50wt% of the total level of antidandruff agent in the composition, more preferably at least 70 wt%.

### Oil-In-Water Emulsion

Compositions according to the invention may comprise an oil in water emulsion.

The aqueous phase of the emulsion contains water. Suitably the emulsion comprises 25 to 85%, preferably from 40 to 70%, more preferably from 45 to 60% water by weight of the total emulsion.

Silicone is present in the oil-in-water emulsion. The silicone is a conditioning agent intended to deposit onto hair remaining behind after rinsing of the hair with water.

The particle size of the silicone (D_{3,2}) droplet within the water in oil emulsion is preferably from 10nm to 10 microns, more preferably having a D_{3,2} mean droplet diameter from 50nm to 5 microns, most preferably from 100nm to 5 microns. D_{3,2} mean droplet diameter may be measured by means of a laser light scattering technique, for example using a 2600D Particle Sizer from Malvern Instruments.

The typical level of the silicone is from 0.1 to 5% by weight of the total composition, preferably from 0.3 to 3 wt%, more preferably from 0.5 to 2.5 wt%.

Typical silicones may include polyalkyl siloxanes, polyaryl siloxanes, polyalkylaryl siloxanes, polyether siloxane copolymer and mixtures thereof.

The silicone may comprises a functionalized silicone. Suitable functionalized silicones include, for example, hydroxyl-, amino-, carboxy-, betaine-, quaternary ammonium-, carbohydrate-, hydroxy- and alkoxy-substituted silicones. The functionalized silicone may also contain multiple substitutions. Preferably, the functionalized silicone is an amino silicone, especiall if the priroctone compound is to be solubilized in t silicone.

Amino silicones are described in EP455185 and include trimethylsilylamodimethicone as depicted below, and are sufficiently water insoluble so as to be useful in the emulsion:

Si (CH₃)₃-O-[Si(CH₃)₂-O-]x-[Si(CH₃) (R₆-NH-CH₂CH₂-NH₂)-O-]y-Si(CH₃)₃

Wherein x+y is a number from about 50 to about 500, and the weight percent amine functionality is from about 0.03% to about 8%, and wherein R₆ is an alkylene group having from 2 to 5 carbon atoms. Preferably, the number x+y is from 100 to 300, and the weight percent amine functionality is from about 0.5% to 4%. As expressed herein, the weight percent amine functionality is measured by titrating a sample of the amino silicone against alcoholic hydrochloric acid to the bromocresol green end point. The weight percent amine is calculated using a molecular weight of 45 (corresponding to CH₃-CH₂-NH₂).

Other cationic polymers include polygalactomannans and polysaccharide polymers, such as cationic cellulose derivatives and cationic starch derivatives.

### Suspending Agent

Suspending agent is a preferred feature of the composition. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2. A suitable heteropolysaccharide gum is xanthan gum, for example that available as Kelzan mu. Mixtures of any of the above suspending agents may be used. Preferred is a mixture of cross-linked polymer of acrylic acid and crystalline long chain acyl derivative.

A most preferred example is a crosslinked polyacrylate polymer.

Suspending agent, if included, will generally be present in a shampoo composition of the invention at levels of from 0.01 to 5 wt.%, preferably from 0.1 to 2.5 wt.%, more preferably from 0.25 to 1 wt.%.

The viscosity of the composition suitably ranges from 3,000 to 10,000 mPa.s, preferably from 4,000 to 8,000 mPa.s, more preferably from 5,000 to 7,000 mPa.s when measured using a Brookfield V2 viscometer (spindle RTV5, 1 minute, 20rpm) at 30°C.

The pH of the composition of the invention preferably ranges from 3 to 9, more preferably from 4 to 7, still more preferably from 4.5 to 6.5.

### Other optional components

The composition may optionally comprise one or more components for use in hair treatment products, provided that the optional components are physically and chemically compatible with the essential components described hereinbefore, and do not otherwise unduly impair sensory, formulation rheology and conditioning performance. Individual concentrations of such optional components may range from 0.001% to 10% by weight of the total composition, preferably from 0.01% to 5%wt%. Such components may include fragrance, dyes, and pigments, pH adjusting agents, pearlescers or opacifiers, viscosity modifiers, preservatives, and natural hair nutrients such as botanicals, fruit extracts, sugar derivatives and amino acids. The composition may comprise an additional silicone which is not in the oil-in-water emulsion phase. The additional silicone may be the same or different from the silicone comprised in the emulsion phase. The composition may also comprise additional antidandruff and/or anti-microbial agents such as pyridinethione salts, pine tar, sulfur, salicylic acid, azoles, selenium sulfide, or mixtures thereof.

### Method of use

The composition is used in a manner for treating a surface. An effective amount of the composition is applied to a desired surface selected from hair and/or scalp, that has been preferably wetted with water. The composition may be allowed to stay on the surface for a given time for it to take effect, preferably combined with massaging, before being rinsed off with water. The given time is preferably from 20 seconds to 2 minutes, more preferably from 30 seconds to 1 minute. The effective amount typically ranges from 1 g to 20g, preferably from 2.5 g to 10g.

The compositions of the invention are primarily intended for topical application to scalp and/or at least a portion of the hair of an individual, either in rinse-off or leave-on compositions, preferably in rinse-off compositions like shampoos.

The following examples are provided to facilitate an understanding of the present invention. Examples according to the invention are illustrated by a number, comparative examples by a letter.

### Examples

The following Examples were prepared.

**Table 1**

| **Example** | **1** | **2** | **3** | **4** | **A** | **B** | **5** | **6** | **7** | **8** | **C** | **D** |
|---|---|---|---|---|---|---|---|---|---|---|---|---|
| | | | | | | | | | | | | |
| Sodium laureth sulphate 1EO | 13 | 13 | 13 | 13 | 13 | 13 | | | | | | |
| Sodium laureth sulphate 3 EO | | | | | | | 8 | 8 | 8 | 8 | 8 | 8 |
| Cocoamidopropyl Betaine | 1.5 | 1.5 | 1.5 | 1.5 | 1.5 | 1.5 | 4 | 4 | 4 | 4 | 4 | 4 |
| Dimethiconol / TEA-dodecylbenzene sulfonate* | 2 | 2 | 2 | 2 | 2 | 2 | 2 | 2 | 2 | 2 | 2 | 2 |
| N-DurHance A1000 (Acrylamidopropyltrimonium Chloride Polymer)** | 0.2 | 0.5 | | | | | 0.2 | 0.5 | | | | |
| N-DurHance AA2000 (Acrylamidopropyltrimonium Chloride / Acrylamide Copolymer)** | | | 0.2 | 0.5 | | | | | 0.2 | 0.5 | | |
| Salcare SC60 (Acrylamidopropyltrimonium Chloride / Acrylamide Copolymer)*** | | | | | 0.2 | 0.5 | | | | | 0.2 | 0.5 |
| Octopirox | 0.5 | 0.5 | 0.5 | 0.5 | 0.5 | 0.5 | 0.5 | 0.5 | 0.5 | 0.5 | 0.5 | 0.5 |
| Water and minors | To 100 | To 100 | To 100 | To 100 | To 100 | To 100 | To 100 | To 100 | To 100 | To 100 | To 100 | To 100 |

| | | | | | | | | | | | | |
|---|---|---|---|---|---|---|---|---|---|---|---|---|
| * molecular weight 200,000, charge density (meq/g) 4.8 ** molecular weight 300,000, charge density ( meq/g) 4.8 *** molecular weight 1,000,000, charge density ( meq/g) 1.9 | | | | | | | | | | | | |

0.1g of Example composition / g hair was applied evenly over a hair switch. Hair switches were washed twice. Once washed, switches were placed in drying cabinet (50°C) until dry. Once dry, the switch was transferred to a jar containing 10ml of ethanol. The Jar was sealed and placed on a bottle roller for known amount of time. Using a syringe, an appropriately sized sample, for analysis, removed from jar, and filtered through a syringe filter into vial for analysis, using HPLC-UV methodologies, against known set of standards containing known amounts of Octopirox in ethanol (µg/ml).

**Table 2 - Octopirox Deposition**

| **Example** | **1** | **2** | **3** | **4** | **A** | **B** | **5** | **6** | **7** | **8** | **C** | **D** |
|---|---|---|---|---|---|---|---|---|---|---|---|---|
| Octopirox deposition (ppm) | 11.93 | 14.6 | 11.01 | 16.63 | 8.61 | 6.56 | 11.91 | 17.13 | 14.32 | 17.68 | 13.19 | 13.64 |
| Std Dev | 2.65 | 3.24 | 2.06 | 8.37 | 1.72 | 0.38 | 2.93 | 1.89 | 1.88 | 3.47 | 2.83 | 1.50 |

The Examples of the invention deposit octopirox onto the hair more effectively than the comparative Examples.

## Claims

1. A hair care composition comprising:
a) piroctone compound selected from the group of compounds comprising piroctone acid, primary, secondary and tertiary olamine salts of piroctone acid, and compounds containing the structure defined by formula (IV): 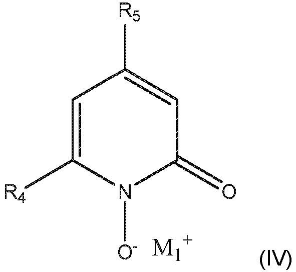 wherein R₄ is selected from C₁-C₁₇ hydrocarbon radicals, R₅ is selected from C1-4 alkyl, C₂₋₄ alkenyl or alkynyl, hydrogen, phenyl or benzyl, and M₁ is selected from hydrogen, monoethanolamine (MEA), diethanolamine (DEA), or triethanolamine (TEA); and
b) cationic homopolymer and/or copolymer comprising an acrylamidopropyltrimonium moiety in which the homopolymer and/or copolymer has a charge density of at least 3.5 meq/g at pH7 and a weight average molecular weight (g/mol) from 100,000 to 500,000 (determined by SEC (Size Exclusion Chromatography) analysis using absolute calibration (universal calibration)).

2. A hair care composition according to any preceding claim in which the weight ratio of homopolymer / copolymer to piroctone compound is 2:1 or below preferably 1.5:1 or below, more preferably from 0.3:1 to 1.1.

3. A hair care composition according to any preceding claim in which the piroctone compound is piroctone olamine.

4. A hair care composition according to any preceding claim in which the copolymer is acrylamidopropyltrimonium chloride/acrylamide copolymer.

5. A hair care composition according to any preceding claim in 4 in which the weight ratio of acrylamidopropyltrimonium chloride to acrylamide within the copolymer is greater than 2:1.

6. A hair care composition according to any preceding claim, in which the level of cationic polymer is from 0.001 to 2% by weight of the total composition.

7. A hair care composition according to any of the preceding claims, wherein the composition is a shampoo.

8. A hair care composition according to any preceding claim comprising an anionic surfactant, preferably an ethoxylated alkyl sulphate anionic surfactant , more preferably sodium laureth sulphate.

9. A hair treatment composition according to any preceding claim, in which the piroctone compound is present at a level of from 0.01 to 5% by weight of the total composition, preferably from 0.05 to 2 wt%, more preferably from 0.1 to 1.5 wt%.

10. A hair treatment composition according to any preceding claim, in which the cationic polymer is present at a level of from 0.01 to 5% by weight of the total composition, preferably from 0.05 to 2 wt%, more preferably from 0.1 to 1 wt%.

11. A hair treatment composition according to any preceding claim further comprising a silicone emulsion.

12. A hair treatment composition according to any preceding in which the piroctone compound is piroctone olamine.

## Patentansprüche

1. Haarpflegezusammensetzung, umfassend:
a) eine Piroctonverbindung, ausgewählt aus der Gruppe von Verbindungen, die Piroctonsäure, primäre, sekundäre und tertiäre Olaminsalze von Piroctonsäure und Verbindungen mit der durch die Formel (IV) definierten Struktur umfassen: 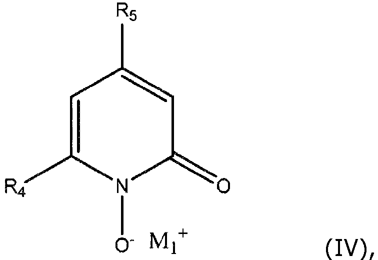
wobei R₄ unter C₁-C₁₇-Kohlenwasserstoffresten ausgewählt ist,
R₅ unter C₁₋₄-Alkyl, C₂₋₄-Alkenyl oder Alkinyl, Wasserstoff, Phenyl oder Benzyl ausgewählt ist und
M₁ unter Wasserstoff, Monoethanolamin (MEA), Diethanolamin (DEA) oder Triethanolamin (TEA) ausgewählt ist; und
b) kationisches Homopolymer und/oder Copolymer, das eine Acrylamidopropyltrimonium-Einheit umfasst, wobei das Homopolymer und/oder Copolymer eine Ladungsdichte von mindestens 3,5 meq/g bei einem pH-Wert von 7 und ein gewichtsmittleres Molekulargewicht (g/mol) von 100.000 bis 500.000 (bestimmt durch SEC(Größenausschluss-chromatographie)-Analyse unter Verwendung einer absoluten Kalibrierung (universelle Kalibrierung) aufweist.

2. Haarpflegezusammensetzung nach dem vorhergehenden Anspruch, wobei das Gewichtsverhältnis von Homopolymer/Copolymer zu Piroctonverbindung 2:1 oder weniger, vorzugsweise 1,5:1 oder weniger, bevorzugter von 0,3:1 bis 1,1 beträgt.

3. Haarpflegezusammensetzung nach einem vorhergehenden Anspruch, wobei die Piroctonverbindung Piroctonolamin ist.

4. Haarpflegezusammensetzung nach einem vorhergehenden Anspruch, wobei das Copolymer Acrylamidopropyltrimoniumchlorid/Acrylamid-Copolymer ist.

5. Haarpflegezusammensetzung nach einem vorhergehenden Anspruch, wobei das Gewichtsverhältnis von Acrylamidopropyltrimoniumchlorid zu Acrylamid innerhalb des Copolymers größer als 2:1 ist.

6. Haarpflegezusammensetzung nach einem vorhergehenden Anspruch, wobei der Gehalt an kationischem Polymer 0,001 bis 2 Gew.-% der Gesamtzusammensetzung beträgt.

7. Haarpflegezusammensetzung nach einem vorhergehenden Anspruch, wobei die Zusammensetzung ein Shampoo ist.

8. Haarpflegezusammensetzung nach einem vorhergehenden Anspruch, umfassend ein anionisches Tensid, vorzugsweise ein anionisches ethoxyliertes Alkylsulfat-Tensid, bevorzugter Natriumlaurethsulfat.

9. Haarbehandlungszusammensetzung nach einem vorhergehenden Anspruch, wobei die Piroctonverbindung in einer Menge von 0,01 bis 5 Gew.-% der Gesamtzusammensetzung, vorzugsweise von 0,05 bis 2 Gew.-%, bevorzugter von 0,1 bis 1,5 Gew.-%, vorhanden ist.

10. Haarbehandlungszusammensetzung nach einem vorhergehenden Anspruch, wobei das kationische Polymer in einer Menge von 0,01 bis 5 Gew.-% der Gesamtzusammensetzung, vorzugsweise von 0,05 bis 2 Gew.-%, bevorzugter von 0,1 bis 1 Gew.-%, vorhanden ist.

11. Haarbehandlungszusammensetzung nach einem vorhergehenden Anspruch, die ferner eine Silikonemulsion umfasst.

12. Haarbehandlungszusammensetzung nach einem vorhergehenden Anspruch, wobei die Piroctonverbindung Piroctonolamin ist.

## Revendications

1. Composition de soins capillaires comprenant :
a) un composé piroctone choisi dans le groupe des composés comprenant l'acide de piroctone, les sels olamine primaires, secondaires et tertiaires de l'acide de piroctone, et les composés contenant la structure définie par la formule (IV) : 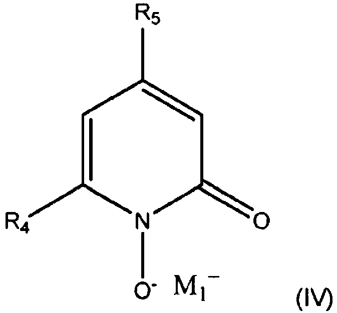 dans laquelle R₄ est choisi parmi les radicaux hydrocarbonés en C₁ à C₁₇, R₅ est choisi parmi un radical alkyle en C1-4, un radical alcényle ou alcynyle en C₂₋₄, un atome d'hydrogène, un radical phényle ou benzyle, et M₁ est choisi parmi un atome d'hydrogène, la monoéthanolamine (MEA), la diéthanolamine (DEA) ou la triéthanolamine (TEA) ; et
b) un homopolymère et/ou copolymère cationique comprenant un fragment acrylamidopropyltrimonium, l'homopolymère et/ou copolymère ayant une densité de charge d'au moins 3,5 méq/g à pH 7 et une masse moléculaire moyenne en masse (g/mol) de 100 000 à 500 000 (déterminée par une analyse SEC (chromatographie par exclusion de taille) à l'aide d'un étalonnage absolu (étalonnage universel)).

2. Composition de soins capillaires selon une quelconque revendication précédente dans laquelle le rapport pondéral homopolymère/copolymère à composé piroctone est inférieur ou égal à 2:1, de préférence inférieur ou égal à 1,5:1, plus préférablement de 0,3:1 à 1,1.

3. Composition de soins capillaires selon une quelconque revendication précédente dans laquelle le composé piroctone est la piroctone olamine.

4. Composition de soins capillaires selon une quelconque revendication précédente dans laquelle le copolymère est un copolymère chlorure d'acrylamidopropyltrimonium/acrylamide.

5. Composition de soins capillaires selon une quelconque revendication précédente en 4 dans laquelle le rapport pondéral chlorure d'acrylamidopropyltrimonium à acrylamide dans le copolymère est supérieur à 2:1.

6. Composition de soins capillaires selon une quelconque revendication précédente, dans laquelle la teneur en polymère cationique est de 0,001 à 2 % en poids de la composition totale.

7. Composition de soins capillaires selon une quelconque revendication précédente, dans laquelle la composition est un shampooing.

8. Composition de soins capillaires selon une quelconque revendication précédente comprenant un tensioactif anionique, de préférence un tensioactif anionique de sulfate d'alkyle éthoxylé, plus préférablement le laureth sulfate de sodium.

9. Composition de traitement capillaire selon une quelconque revendication précédente, dans laquelle le composé piroctone est présent à une teneur de 0,01 à 5 % en poids de la composition totale, de préférence de 0,05 à 2 % en poids, plus préférablement de 0,1 à 1,5 % en poids.

10. Composition de traitement capillaire selon une quelconque revendication précédente, dans laquelle le polymère cationique est présent à une teneur de 0,01 à 5 % en poids de la composition totale, de préférence de 0,05 à 2 %, et plus préférablement de 0,1 à 1 % en poids.

11. Composition de traitement capillaire selon une quelconque revendication précédente comprenant en outre une émulsion de silicone.

12. Composition de traitement capillaire selon une quelconque revendication précédente dans laquelle le composé piroctone est la piroctone olamine.
